# EUROPÄISCHE PATENTANMELDUNG

(11) **EP 2 052 749 A2**
(43) Veröffentlichungstag der Anmeldung: **29.04.2009**
(21) Anmeldenummer: 08016945.1
(22) Anmeldetag: 26.09.2008
(51) Int. Cl.: A61L 24/06, A61L 24/04

(54) **Polymethylmethacrylat-Knochenzement**

(30) Priorität: 22.10.2007 DE 102007050768
(71) Anmelder: Heraeus Medical GmbH, 61273 Wehrheim (DE)
(72) Erfinder: Vogt, Sebastian, Dr., 99092 Erfurt (DE); Büchner, Hubert, Dr., 64354 Reinheim (DE)
(74) Vertreter: Kühn, Hans-Christian

(57) **Zusammenfassung**

Es wird ein PMMA-Knochenzement beschrieben, enthaltend
mindestens ein Homopolymer oder ein Copolymer, das eine Glasübergangstemperatur von größer 45 °C besitzt, und
mindestens ein biokompatibles Elastomer mit einer Glastemperatur kleiner 37 °C, das in Alkylmethacrylaten und/oder Alkyldimethacrylaten und/oder Alkyltrimethacrylaten und/oder Alkyltetramethacrylaten löslich oder unlöslich ist und einen Restmonomergehalt von kleiner 5 Prozent besitzt. Der PMMA-Knochenzement wird vorzugsweise als selbsthärtender Kunststoff zur Fixierung von primären Totalgelenkendoprothesen und von Revisions-Totalgelenkendoprothesen verwendet. Weiterhin kann der PMMA-Knochenzement Verwendung als selbsthärtendes Füllmaterial für die Vertebroplastie, die Kyphoplastie und die Femurhals-Augmentation sowie für die Herstellung von temporären Platzhaltern für zweizeitige Revisionen von Totalgelenkendoprothesen finden.

## Beschreibung

Gegenstand der Erfindung sind Polymethylmethacrylat-Knochenzemente (PMMA-Knochenzemente) mit einer im Vergleich zu konventionellen PMMA-Knochenzementen erhöhten Schlagzähigkeit und Ermüdungsfestigkeit, insbesondere 2-Komponenten PMMA-Knochenzemente mit Pulver- und Flüssigkomponente oder Pasten-Knochenzemente.

PMMA-Knochenzemente sind seit Jahrzehnten bekannt und gehen auf die grundlegenden Arbeiten von Sir Charnley zurück [J. Charnley, J. Bone Joint Surg. 42 (1960) 28-30].

Der Grundaufbau der PMMA-Knochenzemente ist seit dem prinzipiell gleich geblieben. PMMA-Knochenzemente bestehen aus einer flüssigen Monomerkomponente und einer Pulverkomponente [G. Lewis, J. Biomed Mater. Res. (Appl. Biomater.) 38 (1997) 155 - 182]. Die Monomerkomponente enthält im Allgemeinen das Monomer Methylmethacrylat und einen darin gelösten Aktivator (N,N-Dimethyl-p-toluidin). Die Pulverkomponente besteht aus einem oder mehreren Polymeren, die auf Basis von Methylmethacrylat und Comonomeren, wie Styren, Methylacrylat oder ähnlichen Monomeren durch Polymerisation, vorzugsweise Suspensionspolymerisation, hergestellt sind, einem Röntgenopaker und dem Initiator Dibenzoylperoxid. Beim Vermischen der Pulverkomponente mit der Monomerkomponente entsteht durch Quellung der Polymere der Pulverkomponente im Methylmethacrylat ein plastisch verformbarer Teig. Gleichzeitig reagiert der Aktivator N,N-Dimethyl-p-toluidin mit dem Dibenzoylperoxid, das unter Bildung von Radikalen zerfällt. Die gebildeten Radikale initiieren die radikalische Polymerisation des Methylmethacrylates. Mit fortschreitender Polymerisation des Methylmethacrylates erhöht sich die Viskosität des Zementteigs bis der Zementteig erstarrt und damit ausgehärtet ist.

Alternativ zum Pulver-Flüssigkeits-System sind auch 2-Komponenten-Pastenzemente bekannt geworden.

Die grundlegenden mechanischen Anforderungen an PMMA-Knochenzemente, wie 4-Punkt-biegefestigkeit, Biegemodul und Druckfestigkeit, sind in der ISO 5833 beschrieben. Daneben ist die Schlagzähigkeit eine wichtige mechanische Kenngröße der PMMA-Knochenzemente. Diese Eigenschaft charakterisiert die Widerstandsfähigkeit des PMMA-Knochenzementes gegenüber schnellen, mechanischen Krafteinwirkungen. Bei der Erweiterung des Anwendungsgebietes von PMMA-Knochenzementen auf die Kyphoplastie, Vertebroplastie und insbesondere der Femurhals-Augmentation ist die Schlagzähigkeit des PMMA-Knochenzementes von besonderer Bedeutung. Weiterhin steht die Schlagzähigkeit in engem Zusammenhang mit der mechanischen Dauerermüdungsfestigkeit des PMMA-Knochenzementes. Wünschenswert ist deshalb ein PMMA-Knochenzement mit einer erhöhten Widerstandsfähigkeit gegenüber schnellen mechanischen Krafteinwirkungen und einer verbesserten Ermüdungsfestigkeit.

Der Erfindung liegt die Aufgabe zu Grunde, einen PMMA-Knochenzement zu entwickeln, der eine erhöhte Schlagzähigkeit und eine erhöhte Ermüdungsfestigkeit gegenüber konventionellen PMMA-Knochenzementen besitzt, gleichzeitig aber die Mindestanforderungen hinsichtlich der 4-Punktbiegefestigkeit, des Biegemoduls und der Druckfestigkeit erfüllt. Der schlagzähe PMMA-Knochenzement muss zwingend biokompatibel sein.

Die Aufgabe wurde erfindungsgemäß durch Polymethylmethacrylat-Knochenzemente (PMMA-Knochen-zemente) gemäß Anspruch 1 gelöst. Es handelt sich um 2-Komponenten PMMA-Knochenzemente mit Pulver- und Flüssigkomponente oder um Pasten-Knochenzemente.
Die Knochenzemente zeichne sich dadurch aus, dass mindestens ein Homopolymer oder ein Copolymer, das eine Glasübergangstemperatur von mindestens 45 °C besitzt, und mindestens ein biokompatibles Elastomer mit einer Glastemperatur von höchstens 37 °C, das in Alkylmethacrylaten und/oder Alkyldimethacrylaten und/oder Alkyltrimethacrylaten und/oder Alkyltetramethacrylaten löslich oder quellbar ist und einen Restmonomergehalt von kleiner 5 Prozent besitzt, im PMMA-Knochenzement vorhanden sind. Die Kombination eines Homopolymers oder Copolymers mit einer Glastemperatur von mindestens 45 °C mit mindestens einem Elastomer mit einer Glastemperatur höchstens 37 °C ergibt überraschenderweise einen formstabilen PMMA-Knochenzement, der einerseits die mechanischen Mindestanforderungen nach der ISO 5833 erfüllt und andererseits eine deutlich erhöhte Schlagzähigkeit besitzt. Als Homopolymer wird Polymethylmethacrylat bevorzugt und als Copolymere Poly-methylmethacrylat-co-methylacrylat und Poly-methylmethacrylat-co-styren. Im Rahmen der Erfindung sind auch Copolymere, die neben Methylmethacrylat auch aus anderen Alkylmethacrylaten aufgebaut sind. Wesentlich ist außerdem, dass das Elastomer einen Restmonomergehalt von kleiner 5 Prozent haben muss, damit keine toxischen Wirkungen von dem PMMA-Knochenzement ausgehen können.

Das Elastomer liegt bevorzugt im Zementpulver in partikulärer Form mit einer bevorzugten Partikelgröße im Bereich von 5-500 µm vor. Es ist auch im Rahmen der Erfindung möglich, dass das Elastomer in den Partikeln des Hompopolymers oder Copolymers suspendiert ist.

Das Elastomer ist zweckmäßig im Monomer oder Monomergemisch gelöst oder in Form von gequollenen Partikeln im Monomer oder Monomergemisch suspendiert.

Es ist weiterhin möglich, dass das Elastomer in einer Zementpaste gelöst oder in Form von gequollenen Partikeln in der Zementpaste suspendiert ist.

Elastomere mit einer Glastemperatur kleiner 0°C sind bevorzugt.

Als Elastomere kommen besonders Acrylat-Kautschuk, Ethylen-Acrylat-Kautschuk, Ethylen-Propylen-Terpolymer, Ethylen-Vinylacetat-Copolymer, Polybutadien, Polyisopren, Butylkautschuk, Naturkautschuk, Styrol-Butadien-Kautschuk und Polynorbornen in Frage.

Außerdem ist der bevorzugte Elastomergehalt im Polymethylmethacrylat-Knochenzement 0,1-20,0 Masseprozent. Besonders bevorzugt ist ein Elastomergehalt von 2,0-5,0 Masseprozent.

Der erfindungsgemäße PMMA-Knochenzement wird als selbsthärtender Kunststoff verwendet, der zur Fixierung von primären Totalgelenkendoprothesen und von Revisions-Totalgelenkendoprothesen bereitgestellt wird.

Weiterhin findet der erfindungsgemäße PMMA-Knochenzement Verwendung als selbsthärtendes Füllmaterial, das für die Vertebroplastie, Kyphoplastie und zur Femurhals-Augmentation bereitgestellt wird.

Der PMMA-Knochenzement kann auch erfindungsgemäß zur Herstellung von temporären Platzhaltern für zweizeitige Revisionen von Totalgelenkendoprothesen verwendet werden.

Die Erfindung wird durch nachstehende Beispiele näher erläutert, ohne jedoch die Erfindung zu beschränken. Teile und Prozentangaben beziehen sich wie in der übrigen Beschreibung auf das Gewicht, sofern nicht anders angegeben.

Als Referenzmaterial wurde der konventionelle PMMA-Knochenzement Palacos® R eingesetzt.

Es wurden zuerst folgende Zementpulver-Gemische durch zweistündige Vermahlung der nachfolgend aufgeführten Komponenten in einer Porzellan-Kugelmühle hergestellt:

| Beispiel-Nr. | Zusammensetzung des Zementpulvers | | | |
|---|---|---|---|---|
| | Dibenzoylperoxid | Poly-methyl-methacrylat-comethylacrylat | ZrO₂ | Polystyrol-cobutadien |
| Palacos® R | 0,40 g | 33,70 g | 5,90 g | - |
| 1 | 0,39 g | 32,86 g | 5,75 g | 1,00 g |
| 2 | 0,38 g | 32,02 g | 5,60 g | 2,00 g |
| 3 | 0,36 g | 30,33 g | 5,31 g | 4,00 g |

Das Poly-methyl-methacrylat-co-methylacrylat hatte eine Glasübergangstemperatur von 65 °C und das verwendete Poly-styrol-co-butadien (BAYMOND) von kleiner 0 °C. Das Poly-styrol-co-butadien hatte einen gaschromatographisch bestimmten Restmonomergehalt von < 3 %. Es lag in partikulärer Form vor und hatte eine Partikelgröße im Bereich von 63-250 µm.

Danach wurde jeweils 40 g Zementpulver mit 20 ml Methylmethacrylat vermischt, in dem 1,0 Ma% N,N-Dimethyl-p-toluidin gelöst waren. Es bildet sich ein Teig, der in quadratische, flache Hohlformen (Höhe 3 mm) gestrichen wurde und dort nach wenigen Minuten aushärtete. Aus den ausgehärteten Zementplatten wurden Streifen (75 mm x 10 mm x 3 mm) gesägt. Die Biegefestigkeit und die Schlagzähigkeit der Zementstreifen wurden nach der Dynstat-Methode bestimmt.

| Beispiel-Nr. | Biegefestigkeit [MPa] | Schlagzähigkeit [J/m²] |
|---|---|---|
| Palacos® R | 94,7 ± 1,9 | 4,9 ± 0,4 |
| 1 | 91,6 ± 4,4 | 5,5 ± 1,3 |
| 2 | 91,0 ± 3,1 | 7,8 ± 0,7 |
| 3 | 76,5 ± 3,4 | 8,2 ± 1,3 |

Es wurden die 4-Punkt-Biegefestigkeit und das Biegemodul mit Hilfe einer Zwick-Universalprüfmaschine ermittelt. Die Bestimmung der Druckfestigkeit erfolgte mit Hilfe von zylinderförmigen Körpern (h= 12 mm, d= 6 mm). In der ISO5833 wird für PMMA-Knochenzemente im 4-Punktbiegeversuch eine Mindestfestigkeit von ≥ 50 MPa und ein Biegemodul ≥ 1800 MPa sowie eine Mindestdruckfestigkeit von ≥ 70 MPa gefordert.

| Beispiel-Nr. | Druckfestigkeit [MPa] | 4-Punkt-Biegung | |
|---|---|---|---|
| | | Biegefestigkeit [MPa] | Biege-Modul [MPa] |
| Palacos® R | 91,5 ± 3,1 | 65,3 ± 1,9 | 2793 ± 206 |
| 1 | 88,9 ± 2,9 | 59,4 ± 1,1 | 2394 ± 119 |
| 2 | 81,0 ± 3,0 | 54,6 ± 1,5 | 2242 ± 140 |
| 3 | 103,8 ± 3,4* | nicht bestimmbar** | 1963 ± 37 |

| | | | |
|---|---|---|---|
| * Probekörper verformt, kein Bruch ** Probekörper sind nicht gebrochen, Probekörper wurden nur deformiert | | | |

Die in der Tabelle dargestellten Ergebnisse zeigen, dass die mechanischen Mindestanforderungen nach der ISO5833 von den PMMA-Knochenzementen der Beispiele 1 und 2 erfüllt wurden. Beim Beispiel 3 ist auch davon auszugehen, obwohl kein Bruch der Probekörper erfolgte.

Zylinderförmige Probekörper von Palacos® R und den Zementen der Beispiele 1 und 2 wurden hinsichtlich der in vitro-Cytotoxizität entsprechend der ISO10993-5 geprüft. Die Probekörper zeigten keine cytotoxische Eigenschaften unter den angewandten Untersuchungsbedingungen.

Es wurde weiterhin 2,0 g mit Methacrylat-Gruppen terminiertes Poly-styrol-co-butadien in 20 ml Methylmethacrylat gelöst, das 1,0 % N,N-Dimethyl-p-toluidin enthielt. Diese Monomerlösung wurde mit 40,0 g Zementpulver vermischt, das 0,4 g Dibenzoylperoxid, 33,7 g Poly-methyl-methacrylat-co-methylacrylat und 5,9 g Zirkoniumdioxid enthielt. Es bildete sich ein Zementteig, der nach ungefähr 8 Minuten aushärtete. Die Schlagzähigkeit war vergleichbar mit der des Beispiels 2.

Es wurde zusätzlich das Ermüdungsverhalten des PMMA-Knochenzementes des Beispiels 1 untersucht. Dazu wurden Streifen (75 mm x 10 mm x 3 mm) hergestellt und für 4 Wochen in destilliertem Wasser bei 37 °C gelagert. Es wurde anschließend bei drei Lastniveaus die Dauerlastwechselfestigkeit bestimmt, wobei die Frequenz 5 Hz betrug. In der nachfolgenden Abbildung ist die Wöhlerkurve des untersuchten PMMA-Zementes des Beispiels 1, hierbei bezeichnet als P-Zement, im Vergleich zur Wöhler-Kurve des Palacos® R dargestellt.

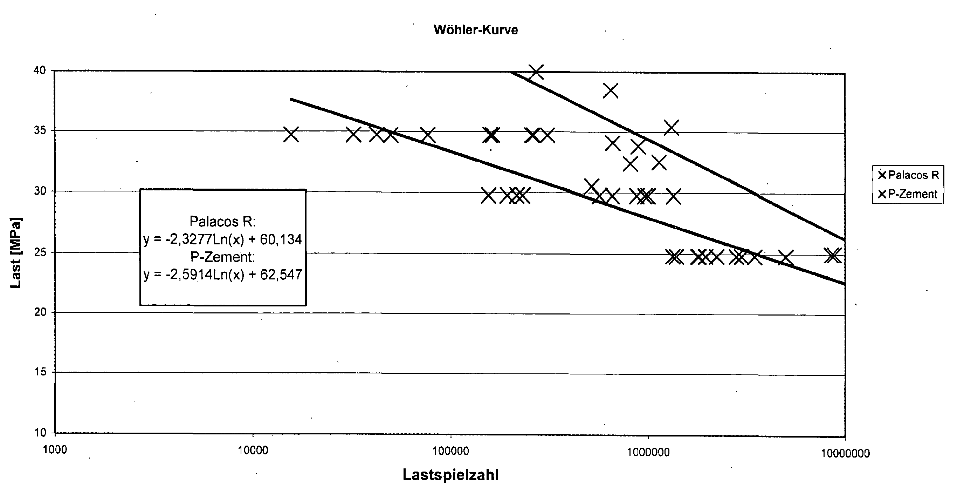

## Patentansprüche

1. 2-Komponenten PMMA-Knochenzement mit Pulver- und Flüssigkomponente, oder Pasten-Knochenzement,
enthaltend
**A** mindestens ein Homopolymer oder ein Copolymer, das eine Glasübergangstemperatur von mindestens 45 °C besitzt, und
**B** mindestens ein biokompatibles Elastomer mit einer Glastemperatur von höchstens 37 °C, das in Alkylmethacrylaten und/oder Alkyldimethacrylaten und/oder Alkyltrimethacrylaten und/oder Alkyltetramethacrylaten löslich oder unlöslich ist und einen Restmonomergehalt von höchstens 5 Prozent besitzt.

2. PMMA-Knochenzement nach Anspruch 1, **dadurch gekennzeichnet, dass** das Elastomer bevorzugt in der Pulverkomponente in partikulärer Form mit einer bevorzugten Partikelgröße im Bereich von 5-500 µm vorliegt.

3. PMMA-Knochenzement nach einem der Ansprüche 1 und 2, **dadurch gekennzeichnet, dass** das Elastomer im Monomer oder Monomergemisch gelöst ist oder in Form von gequollenen Partikeln im Monomer oder Monomergemisch suspendiert ist.

4. PMMA-Knochenzement nach einem der Ansprüche 1-3, **dadurch gekennzeichnet, dass** das Elastomer in einer Zementpaste gelöst ist oder in Form von gequollenen Partikeln in der Zementpaste suspendiert ist.

5. PMMA-Knochenzement nach mindestens einem der Ansprüche 1-4, **dadurch gekennzeichnet, dass** das Elastomer eine Glastemperatur kleiner 0°C besitzt.

6. PMMA-Knochenzement nach mindestens einem der Ansprüche 1-5, **dadurch gekennzeichnet, dass** das Elastomer der Gruppe bestehend aus Acrylat-Kautschuk, Ethylen-Acrylat-Kautschuk, Ethylen-Propylen-Terpolymer, Ethylen-Vinylacetat-Copolymer, Polybutadien, Polyisopren, Butylkautschuk, Naturkautschuk, Styrol-Butadien-Kautschuk und Polynorbornen entstammt.

7. PMMA-Knochenzement nach mindestens einem der Ansprüche 1-6, **dadurch gekennzeichnet, dass** das Elastomer im Polymethylmethacrylat-Knochenzement zu 0,1 bis 20,0 Masseprozent vorliegt.

8. PMMA-Knochenzement nach Anspruch 7, **dadurch gekennzeichnet, dass** das Elastomer im Polymethylmethacrylat-Knochenzement zu 2,0 bis 5,0 Masseprozent vorliegt.

9. Verwendung eines PMMA-Knochenzements nach einem der Ansprüche 1-8 zur Herstellung eines Mittels zur Fixierung von primären Totalgelenkendoprothesen und von Revisions-Totalgelenkendoprothesen.

10. Verwendung eines PMMA-Knochenzements nach einem der Ansprüche 1-7 zur Herstellung eines Füllmaterials für die Vertebroplastie, Kyphoplastie oder Femurhals-Augmentation.

11. Verwendung eines PMMA-Knochenzements nach einem der Ansprüche 1-7 zur Herstellung von temporären Platzhaltern verwendet wird.
